# FASCICULE DE BREVET EUROPEEN

(11) **EP 0 959 780 B1**
(45) Date de publication et mention de la délivrance du brevet: **31.03.2004**
(21) Numéro de dépôt: 97952042.6
(22) Date de dépôt: 08.12.1997
(51) Int. Cl.: A61B 17/15, A61F 2/46

(54) **ANCILLAIRE POUR LA PREPARATION DE LA POSE D'UNE PROTHESE DE GENOU**
HILFSINSTRUMENT ZUR VORBEREITUNG DES EINSETZENS EINER KNIEPROTHESE
ANCILLARY EQUIPMENT FOR PREPARING THE SETTING OF A KNEE PROSTHESIS

(30) Priorité: 10.12.1996 BE 9601031
(43) Date de publication de la demande: 01.12.1999
(73) Titulaire: Memento S.A., 1217 Meyrin 1 - Genève (CH)
(72) Inventeur: LOOTVOET, Louis, B-5000 Namur (BE); MULLIER, Jean, B-1360 Orbais (BE)
(74) Mandataire: Jacobson, Claude
(86) Numéro de dépôt international: PCT/EP1997/006833
(87) Numéro de publication internationale: WO 1998/025526

(56) Documents cités:
- EP-A- 0 327 249
- EP-A- 0 720 834
- WO-A-95/13034
- DE-U- 9 301 611
- FR-A- 2 681 778

## Description

La présente invention concerne d'une manière générale les prothèses de genou et plus particulièrement la préparation de la pose d'une prothèse de genou au moyen d'un matériel approprié.

La pose d'une prothèse de genou peut être effectuée soit en restituant les épaisseurs osseuses sans se préoccuper des ligaments, soit, en restituant l'équilibre ligamentaire donc la cinématique, par rétablissement d'une bonne tension des ligaments. D'une manière générale on cherche à obtenir un bon équilibre en flexion-extension afin qu'il n'y ait pas, une fois la prothèse posée, de laxité dans un sens ou dans l'autre, ce qui nécessite que soit assuré cet équilibre.

Le chirurgien pratique donc d'abord une coupe de l'extrémité proximale du tibia, orthogonale à l'axe mécanique du tibia, lequel est le plus souvent confondu avec l'axe anatomique ou centro-médullaire du tibia. Cette coupe tibiale ne soulève pas de problème particulier, des repères anatomiques permettant d'exécuter aisément cette coupe avec une épaisseur prédéterminée.

Quant à la coupe de l'extrémité distale du fémur, elle doit être orthogonale à l'axe mécanique du fémur, lequel est incliné d'un certain angle par rapport à l'axe anatomique du fémur. On pratique cette coupe par exemple sur une épaisseur de 8mm, qui correspond à l'épaisseur de l'implant fémoral.

Puis le chirurgien mesure l'écart d'extension entre les deux plans de coupe du tibia et du fémur au moyen d'un espaceur. Il est possible que cet écart ne soit pas rectangulaire comme il doit l'être, c'est-à-dire que les plans de coupe du tibia et du fémur ne soient pas parallèles. Dans ce cas le chirurgien réalise un allongement des éléments capsulo-ligamentaires rétractés de la concavité, afin d'obtenir un parallélisme entre les extrémités distale du fémur et proximale du tibia.

A ce stade selon la technique antérieure, le chirurgien doit pratiquer les coupes fémorales antérieure et postérieure.

Selon une première méthode, on utilise une référence antérieure sur le fémur. On mesure la distance entre le plan de coupe antérieur A et le plan bicondylien postérieur P, et on prend l'implant fémoral dont la dimension antéro-postérieure AP est égale ou immédiatement inférieure à la valeur relevée. Le chirurgien exécute alors la coupe antérieure selon le plan de référence antérieure A. Il reporte ensuite la dimension AP précédemment choisie pour l'implant et effectue la coupe postérieure. Il obtient ainsi l'écart de flexion FG, qui doit être égal à l'écart d'extension EG pour que la cinématique du genou soit correcte.

Deux hypothèses se présentent alors à ce stade :
a) la dimension AP de la prothèse est égale à la dimension AP du fémur. Dans ce cas aucun problème n'apparaît.
b) la dimension AP de la prothèse est inférieure à la dimension AP du fémur. De ce fait l'écart en flexion FG est supérieur à l'écart d'extension EG, ce qui provoque une laxité en flexion. Pour remédier à cet inconvénient, la technique utilisée jusqu'à présent prévoit de reprendre la coupe distale de l'épaisseur e = (FG-EG). Mais cette opération de recoupe est peu précise. En outre elle impose au chirurgien de mettre en place un plateau tibial en polyéthylène plus épais.

Selon une deuxième méthode connue, classiquement appelée « référence postérieure » telle que décrite par le brevet EP 0 327 249, après les coupes fémorale distale et tibiale proximale, le chirurgien effectue les opérations suivantes :

Il reporte la valeur FG = EG et réalise la coupe postérieure de l'extrémité distale du fémur. Puis il reporte la dimension AP diminuée de l'épaisseur postérieure de l'implant (distance entre les plans de coupe antérieure et postérieure), et réalise la coupe antérieure du fémur. A ce stade trois nouvelles éventualités peuvent se présenter :
a) la dimension AP de l'implant est égale à la dimension AP du fémur. Dans ce cas il n'apparaît aucun problème.
b) si la dimension AP de l'implant est supérieure à la dimension correspondante du fémur, ce dernier est non conforme dans le plan antérieur car il existe un espace entre la partie haute de la trochlée de l'implant et la surface du fémur, ce qui n'est pas satisfaisant.
c) si la dimension AP de l'implant est inférieure à la valeur correspondante du fémur, la trochlée de l'implant se situe dans la corticale antérieure, ce qui peut être à l'origine d'un risque de fracture fémorale.

Le document EP 0 327 249 décrit ainsi une méthode selon laquelle on pratique d'abord l'équilibrage ligamentaire en extension, puis la coupe distale ; enfin on reporte l'écart d'extension en flexion de telle sorte que les deux écarts soient égaux, et on pratique la coupe postérieure (Fig.51).

Ainsi en dehors du cas où la dimension AP de l'implant est égale à la dimension correspondante du fémur, aucune des solutions mises en oeuvre jusqu'à présent ne s'est révélée réellement satisfaisante.

Par ailleurs, le brevet EP précité met en oeuvre un ancillaire à tige solidaire d'une forme en U adaptée pour s'emboîter sur une coulisse complémentaire d'un distracteur. Ce matériel de conception modulaire, génère des jeux constitutifs d'une imprécision angulaire peu compatible avec l'ordre de grandeur des angles à respecter (entre 3 et 11 degrés habituellement).

L'invention a donc pour but de proposer un ancillaire spécifique pour définir un mode opératoire permettant de résoudre ce problème de manière entièrement satisfaisante, en évitant en particulier de devoir recourir à une seconde coupe distale d'ajustement sur le fémur.

Cet ancillaire comprend une tige centro-médullaire associée à un moyen permettant l'obtention d'un équilibre ligamentaire.

L'invention a pour objet un ancillaire pour la préparation de la pose d'une prothèse fémorale de genou comportant un plateau tibial et un implant fémoral, cet ancillaire comprenant une tige centro-médullaire associée à un moyen permettant l'obtention d'un équilibre ligamentaire, caractérisé en ce qu'il comprend une plaque ayant une épaisseur au plus égale à celle d'un plateau tibial de la prothèse de genou, et à laquelle est fixée la tige suivant une inclinaison appropriée pour permettre de restituer l'interligne fémoral ou interligne virtuel avant usure de l'articulation, cette plaque étant destinée à venir prendre appui sur un plan de coupe tibial après insertion de sa tige dans le canal médullaire du fémur afin de permettre l'obtention d'un équilibre ligamentaire et en vue d'un réglage ultérieur des écartements en extension entre la coupe proximale du tibia et l'extrémité distale du fémur, et en flexion entre la coupe proximale du tibia et la coupe postérieure du fémur, et en ce qu'il est pourvu d'une cale adaptable sur ou pouvant être glissée en dessous de la plaque et d'épaisseur variable, afin que l'épaisseur totale de la plaque et de la cale soit égale à la distance entre le plan de coupe proximal tibial et l'extrémité distale du fémur la plus proche dudit plan de coupe, après obtention de l'équilibre ligamentaire par distraction de l'articulation entre la plaque et l'extrémité distale du fémur.

Le matériel selon l'invention permet de mettre en place un plan de référence de coupe distale du fémur, perpendiculaire à l'axe mécanique du membre inférieur. Cet élément permet également. d'obtenir l'équilibre ligamentaire sans pratiquer une coupe fémorale préalable, comme cela est le cas dans la technique antérieure rappelée ci-dessus.

Un second avantage essentiel de ce matériel est qu'il permet de pratiquer la coupe de l'extrémité distale du fémur sans avoir à replacer le membre en extension comme dans la méthode antérieure exposée précédemment.

D'autres particularités et avantages de l'invention apparaîtront au cours de la description qui va suivre, faite en référence aux dessins annexés qui en illustrent une forme de réalisation à titre d'exemple non limitatif.
La figure 1 est une vue schématique en élévation prise dans le plan frontal d'une forme de réalisation du matériel selon l'invention et d'un fémur ainsi que de l'extrémité supérieure du tibia correspondant, la tige du matériel étant insérée dans le canal médullaire du fémur.
La figure 2 est une vue analogue à la figure 1 dans le plan frontal montrant le fémur en position d'extension sur le tibia et la plaque du matériel en appui sur le plan de coupe tibial.
La figure 3 est une vue en coupe suivant 3-3 de la figure 4 analogue aux figures 1 et 2 dans le plan frontal en extension, représentant le matériel muni d'une cale complémentaire d'épaisseur insérée entre l'extrémité supérieure du tibia et l'extrémité distale du fémur.
La figure 4 est une vue suivant 4-4 de la figure 3.
La figure 5 est une vue schématique en élévation dans le plan sagittal montrant le fémur en flexion sur le tibia et muni d'un appareil de mesure de la distance entre le plan de référence antérieure et le plan bicondylien postérieur du fémur pour le choix de la dimension AP de l'implant correspondant.
La figure 6 est une vue schématique dans le plan sagittal montrant un bloc de coupe fémoral dans les plans antérieur et postérieur précédemment déterminés ainsi qu'un palpeur associé, le fémur restant en flexion.
La figure 7 est une vue schématique en élévation dans le plan sagittal montrant un espaceur inséré entre le fémur et le tibia, le fémur étant en flexion, afin de mesurer l'écart de flexion vrai.
La figure 8 est une vue en élévation schématique dans le plan sagittal illustrant la réalisation de la coupe distale du fémur au moyen d'un appareil approprié, le fémur étant toujours en flexion.
La figure 9 est une vue en élévation dans le plan frontal d'une forme de réalisation de l'élément d'ancillaire apparaissant aux figures 1 à 8.
La figure 10 est une vue en perspective partielle de l'élément d'ancillaire de la figure 9.
La figure 11 est une vue en perspective éclatée d'une seconde forme de réalisation de l'élément d'ancillaire ainsi que de la cale associée.
La figure 12 est une vue en élévation latérale de l'élément d'ancillaire et de la cale de la figure 11.
La figure 13 est une vue en perspective éclatée d'un mode de réalisation industrielle de l'appareil de mesure schématisé à la figure 5 pour la mesure de la distance fémorale antéro-postérieure de l'implant et de fixation de la rotation par rapport à la coupe proximale tibiale.
La figure 14 est une vue en coupe longitudinale partielle du dispositif de support et de blocage de la tige-plaque de la figure 13.
La figure 15 est une vue en perspective éclatée à échelle réduite d'un mode de réalisation industrielle de l'appareil schématiquement représenté aux figures 6 et 8 pour la coupe distale du fémur.
La figure 16 est une vue en perspective à échelle réduite d'un mode de réalisation de l'espaceur illustré schématiquement à la figure 7.
La figure 17 est une vue en perspective à échelle réduite d'un bloc de coupe de chanfreins de l'extrémité distale du fémur.
La figure 18 est une vue en élévation frontale du côté intérieur d'une variante du bloc de coupe de la figure 17.
La figure 19 est une vue de dessus du bloc de coupe des figures 17 et 18.
La figure 20 est une vue en élévation schématique dans le plan frontal correspondant à la figure 6.

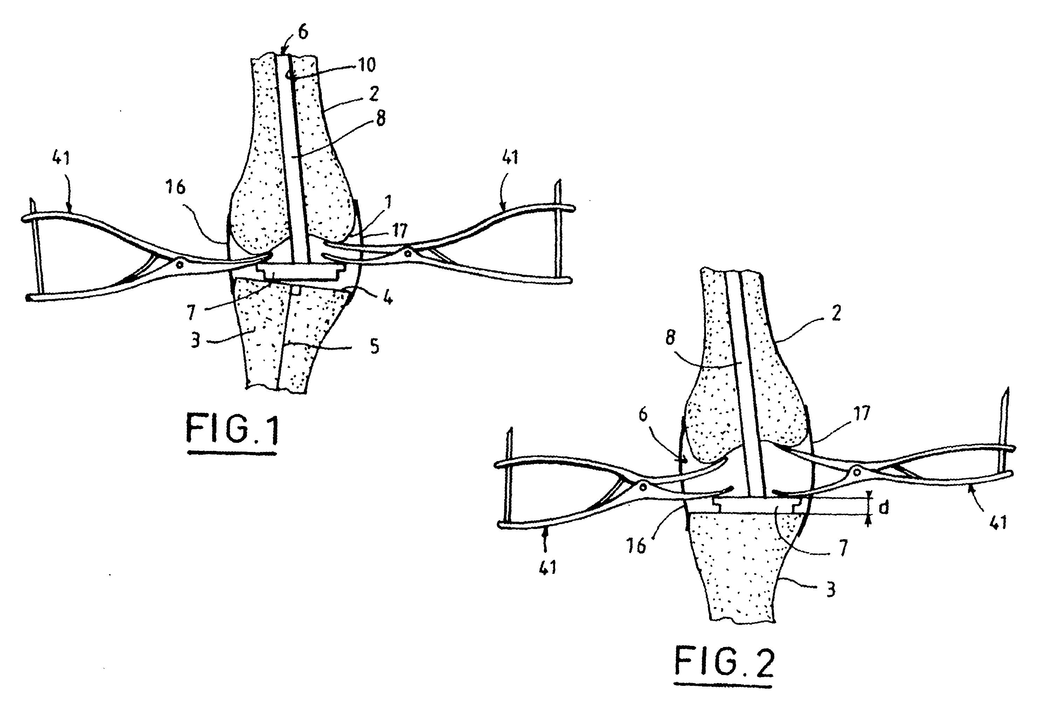

On voit à la figure 1 l'extrémité distale 1 d'un fémur 2 et l'extrémité supérieure d'un tibia 3 relié au fémur 2 par des ligaments interne 16 et externe 17. Sur l'extrémité tibiale on a pratiqué une coupe définissant un plan de coupe tibial 4, perpendiculaire à l'axe mécanique 5 du tibia. Un élément d'ancillaire 6 comportant une plaque 7 et une tige 8 solidaire de la plaque 7, avec laquelle elle forme un ensemble monobloc, est disposé dans l'articulation entre le tibia 3 et le fémur 2, avec sa tige 8 insérée dans le canal médullaire 10 du fémur 2.

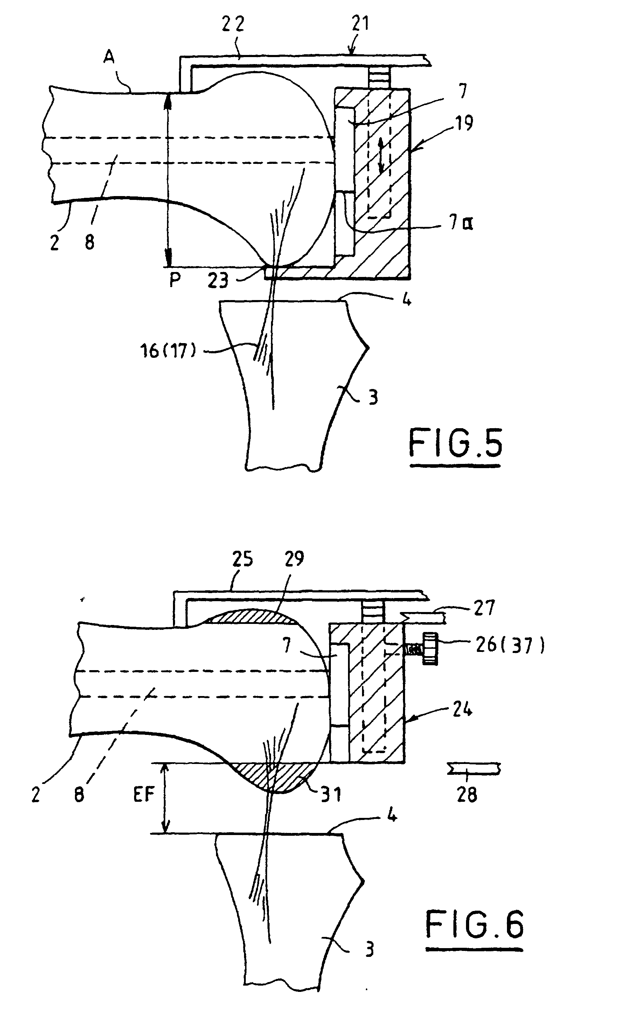

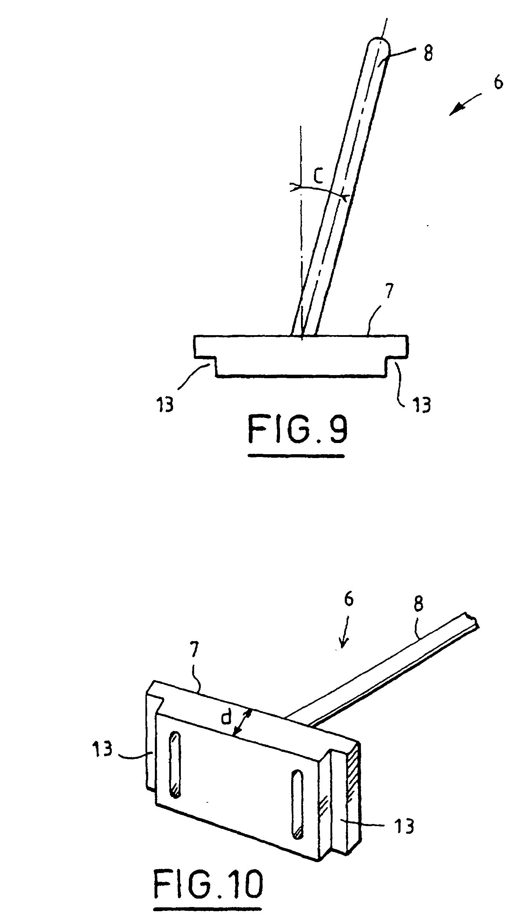

La tige 8 est inclinée sur la surface de la plaque 7 dans le plan frontal d'un angle C approprié en fonction de l'anatomie du patient, le chirurgien disposant d'un jeu d'éléments de matériels 6 à cet effet. La tige 8 est perpendiculaire à la plaque 7 dans le plan sagittal (figure 5). L'angle C (figure 9) peut être compris entre 3 degrés et 11 degrés environ. Le chirurgien choisit ainsi l'ancillaire approprié dans un jeu dont les tiges 8 ont un angle C variable dans ces limites, par exemple par pas de 2 degrés (3°, 5°, 7°, 9°, 11°). La tige 8 peut avoir un diamètre par exemple de 10mm environ, et la plaque 7 une épaisseur de 5mm environ.

Quant à la plaque 7 son épaisseur d est au plus égale à celle du plateau tibial de la prothèse de genou qui doit être posée ultérieurement, cette prothèse étant d'un type connu en soi et non représentée.

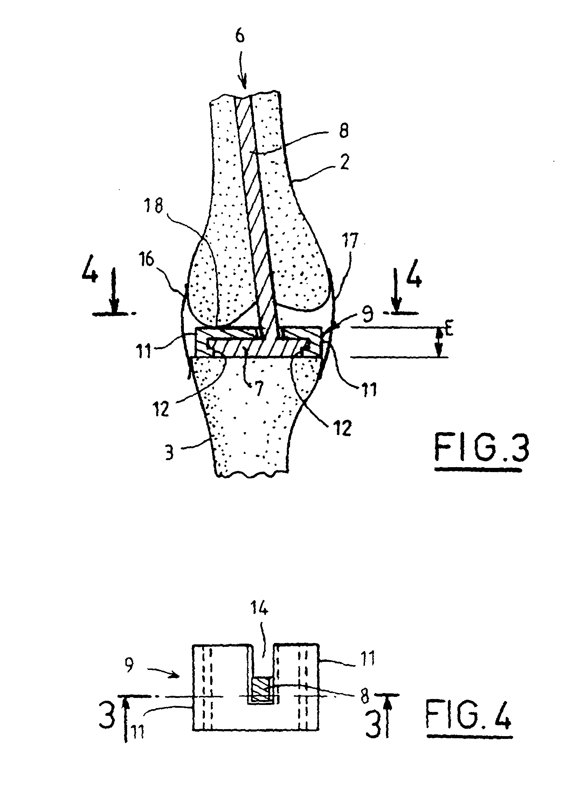

Le matériel 6 est complété par une cale 9 (figures 3 et 4) adaptable sur la plaque 7 et d'épaisseur variable. A cet effet la cale 9 est munie de moyens de solidarisation amovibles avec la plaque 7. Dans l'exemple représenté ces moyens sont constitués par la réalisation de la cale 9 suivant un profil en U emboîtable sur la plaque 7, et dont les deux branches opposées 11 sont prolongées par des pattes transversales 12 venant se loger dans des dégagements conjugués 13 formés sur les côtés de la plaque 7.

La cale 9 présente un évidement médian 14 pratiqué à partir de l'un de ses côtés et s'étendant parallèlement aux pattes 12. Cet évidement 14 reçoit la tige 8 et permet donc de faire coulisser la cale 9 sur la plaque 7 après emboîtement des pattes dans les dégagements 13.

La préparation des coupes fémorales antérieure, postérieure et distale du fémur 2, au moyen de l'ensemble 6 et de sa cale d'épaisseur 9, s'effectue de la manière suivante.

L'ensemble 6 est mis en place comme représenté à la figure 1 avec sa tige 8 dans le canal médullaire 10 du fémur 2. Après distraction à l'aide des pinces 41 de distraction ou de tout autre système extenseur, on constate dans cette position initiale qu'il n'y a pas d'équilibre ligamentaire, la plaque 7 n'étant pas confondue avec le plan de coupe proximal tibial 4, le ligament externe 16 étant plus court que le ligament interne 17, ou vice-versa.

Le chirurgien effectue alors un allongement des éléments capsulo-ligamentaires rétractés de la concavité, ce qui crée un allongement ou réaxation du membre, appelé "release", en redressant le fémur 2 pour le placer dans la position d'extension de la figure 2 au moyen des distracteurs 41, dans laquelle la plaque 7 vient par distraction prendre appui sur le plan de coupe tibial 4. Dans cette position, le contact de la plaque 7 avec le plan de coupe tibial 4 assure un équilibre ligamentaire effectif quelle que soit l'usure fémorale et sans autres moyens de référence que le canal médullaire du fémur.

Le chirurgien met ensuite en place la cale 9 sur la plaque 7 en l'emboitant par ses bords 11 sur les côtés de celle-ci, tandis que la tige 8 prend place dans l'évidement 14 (figures 3 et 4). L'épaisseur de la cale 9 est choisie par le chirurgien après une estimation consécutive à des essais. L'épaisseur de cette cale peut ainsi varier de 2mm en 2mm, la meilleure cale 9 permettant d'obtenir une bonne tension des ligaments 16 et 17 par contact de sa surface supérieure avec la partie distale 18 la plus proche du fémur 2. A ce stade, l'épaisseur totale E de la cale 9 est égale à la distance entre le plan 4 de la coupe proximale tibiale et la partie distale 18 du fémur 2 la plus proche de la coupe tibiale 4. La cale 9 permet donc de déterminer l'épaisseur totale E.

Dans l'étape suivante, représentée à la figure 5, le chirurgien retire la cale 9 puis fait passer le fémur 2 en flexion à 90°.

La plaque 7 comporte une face latérale 7a perpendiculaire à sa face distale utilisée pour venir en appui sur le plan de coupe tibiale 4. Cette face latérale 7a constitue une surface de référence pour le plan de coupe tibial 4, auquel elle doit être disposée parallèlement dans le plan frontal (Fig.5-6).

Le chirurgien procède à une rotation de l'ensemble 6 pour que la face latérale inférieure 7a de la plaque 7 soit parallèle au plan de coupe tibiale 4 dans le plan frontal, puis il monte un bloc 19 sur la plaque 7. Ce bloc 19 fait partie d'un appareil 21 comportant un bras 22 prenant appui sur la corticale antérieure, et une patte 23 qui vient s'appuyer sur la corticale postérieure. L'appareil 21 est connu en soi et ne sera donc pas davantage décrit. Il permet de mesurer l'écart antéro-postérieur AP entre les extrémités des bras 22 et 23, c'est-à-dire l'écartement antéro-postérieur fémoral, afin de déterminer la dimension correspondante de l'implant fémoral.

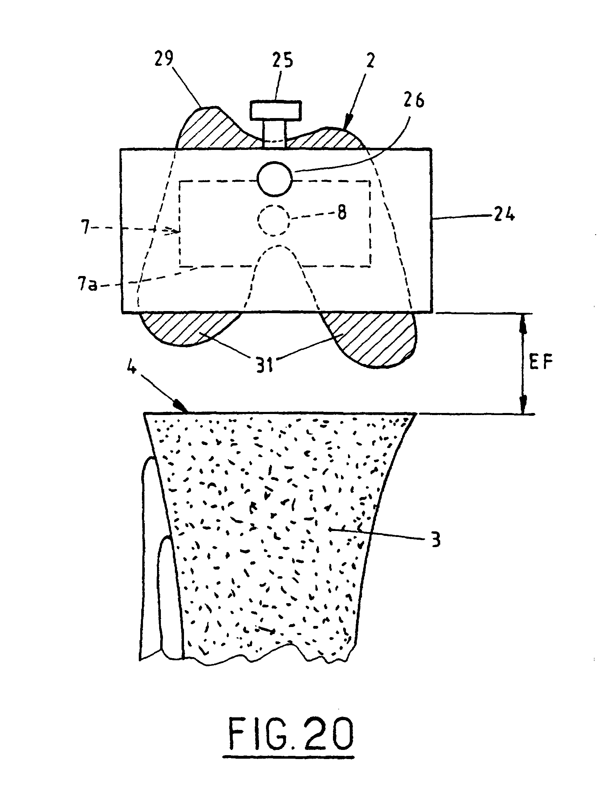

A l'étape suivante, illustrée aux figures 6 et 20, le chirurgien a enlevé l'appareil de mesure 21 et met en place sur la plaque 7 un bloc de coupe 24 adapté pour coulisser sur la plaque 7. Ce bloc 24, réalisé de manière connue en soi, est muni d'un palpeur 25 de référence pouvant prendre appui sur le plan antérieur A de référence pour la coupe à effectuer. Le palpeur 25 est réglable de manière connue en soi par l'intermédiaire d'un bouton de commande 26.

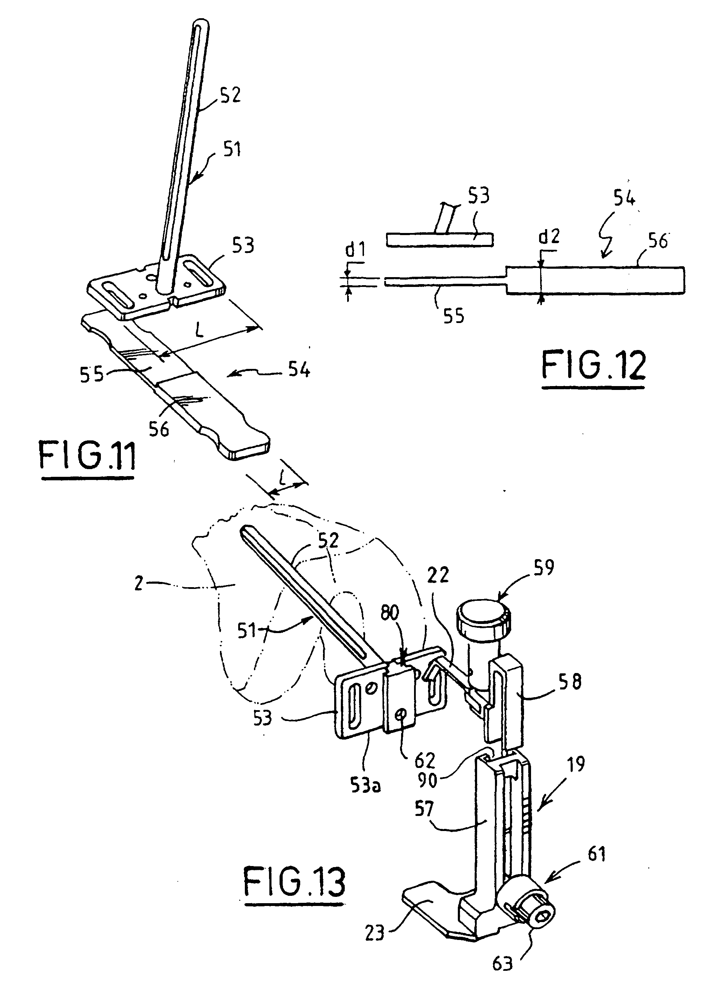

Le chirurgien fixe la rotation du bloc de coupe 24, de telle sorte que la face inférieure de ce bloc soit parallèle à la coupe proximale tibiale 4. (Un mode de réalisation de ces moyens de fixation en rotation est illustré à la figure 13).

Le chirurgien procède à la coupe antérieure de la partie 29 au moyen de la lame 27 en prenant appui sur la face antérieure du bloc 24, et de même avec la lame 28 il prend appui sur la face postérieure du bloc 24 pour procéder à la coupe de la partie postérieure 31. Les caractéristiques de l'implant choisi déterminent la distance entre les plans de coupe antérieure et postérieure.

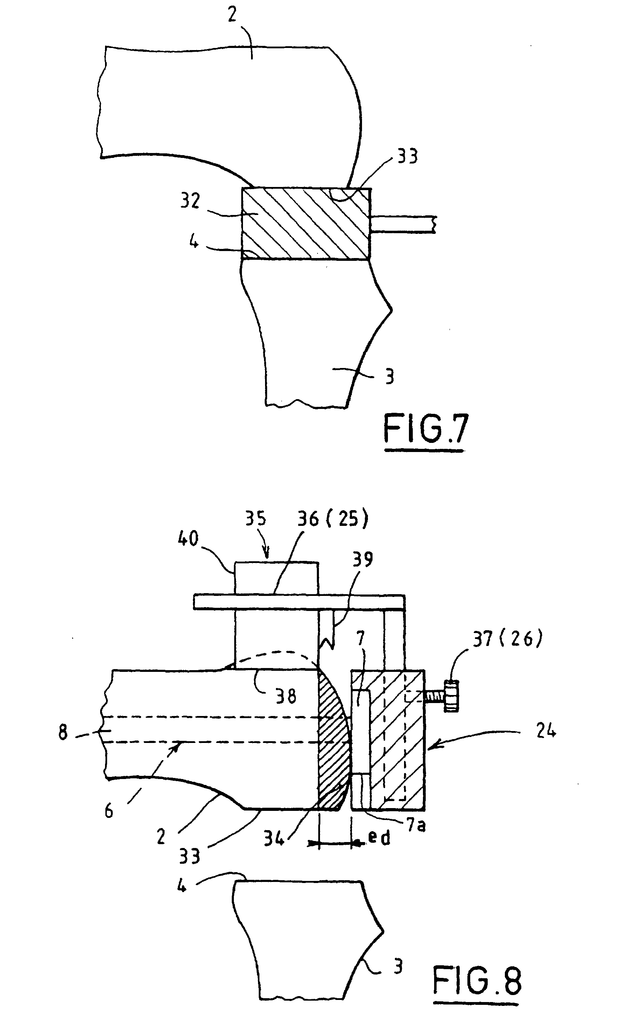

La distance EF entre le plan de coupe tibial 4 et le plan de coupe postérieur P de la partie 31 par la lame 28 est l'écart de flexion vrai. Cet écart de flexion vrai EF peut ensuite être exactement mesuré au moyen d'un espaceur 32 (figure 7) inséré entre le plan de coupe tibial 4 et le plan de coupe postérieure 33. E est comme déjà indiqué la distance entre le plan de coupe tibial 4 et la partie distale 18 du fémur 2 la plus proche. On détermine ed, qui est l'épaisseur de la coupe distale à effectuer, par la relation ed = EF - E afin d'assurer un bon fonctionnement de la prothèse à mettre en place par l'égalité et la rectangularité des écarts d'extension et de flexion.

Enfin il reste au chirurgien à procéder à la coupe de la partie distale 34 (figure 8) d'épaisseur ed. A cet effet le chirurgien met en place un dispositif 35 d'un type connu en soi et qui ne nécessite pas de description détaillée. Ce dispositif 35 comporte un bras latéral 36 réglable en position au moyen d'un bouton de commande 37 et qui est équipé d'un bloc de coupe distale 40. Ce bloc 40 coulisse sur le bras support 36 et peut venir prendre appui sur le plan de coupe antérieur 38. Une lame 39 dont la position est réglable permet d'exécuter la coupe de la partie 34 suivant l'épaisseur voulue ed.

Après quoi le chirurgien peut enlever le dispositif 35 et extraire l'ensemble 6 du fémur 2. En vue de la pose de la prothèse de genou des chanfreins peuvent alors être réalisés si la géométrie de l'implant le justifie.

Dans le second mode de réalisation illustré à la figure 11, l'élément d'ancillaire 51 comprend une tige 52, une plaque rectangulaire 53 fixée à une extrémité de la tige 52, et une cale 54 de forme allongée, approximativement rectangulaire. La cale 54 a une largeur I sensiblement égale à la moitié de la longueur L de la plaque rectangulaire 53, de manière à n'occuper que sensiblement une moitié de la surface de la plaque 53 lorsque cette cale 54 est glissée au-dessous. La cale 54 comporte une partie antérieure 55 d'épaisseur d1 inférieure à l'épaisseur d2 de sa partie postérieure 56, et qui peut être glissée entre la plaque 54 et le plan de coupe tibiale 4.

L'épaisseur d1 est variable, afin que l'épaisseur totale E de la plaque 53 et de la partie antérieure 55 de la cale 54 soit égale à la distance entre le plan de coupe proximal tibial 4 et l'extrémité distale 18 du fémur 2 la plus proche dudit plan de coupe, après obtention de l'équilibre ligamentaire par distraction de l'articulation entre la plaque 53 et l'extrémité distale du fémur.

Par rapport à la cale 9 de la figure 3, la cale 54 présente l'avantage de n'occuper que la partie gauche de la figure 3 pour la partie distale 18 la plus longue du fémur 2 servant à la mesure de l'écart en extension E. Grâce à cette libération de la partie droite de la plaque 7, située de l'autre côté de la tige 8, il est possible d'y loger un distracteur, ce qui était difficile à réaliser antérieurement en raison du manque d'espace disponible. La cale allongée 54 est plus aisée à manipuler et à mettre en place entre la plaque 53 et le plan de coupe tibiale 4, également par le fait qu'il n'est pas nécessaire de l'emboîter sur des profils latéraux correspondants de la plaque 53. Sa partie plus épaisse 56 sert de patte de manipulation pour le chirurgien.

Les dispositifs illustrés aux figures 13 à 16 sont des modes de réalisation industrielle des appareils représentés schématiquement aux figures 5 et 8.

La figure 13 montre le bloc 19, constitué d'un montant 57 profilé en U, dans lequel peut coulisser un support 58 du bras 22, la position horizontale de ce dernier par rapport au support 58 étant réglable au moyen d'un dispositif 59 connu en soi. Sur le montant 57 peut également coulisser un système de blocage 61 de la position en hauteur de la tige-plaque 51. A cet effet la plaque 53 est percée d'un trou 62 de réception d'une vis 63 pouvant traverser axialement le système de blocage 61 pour relier ce dernier à l'élément d'ancillaire 51.

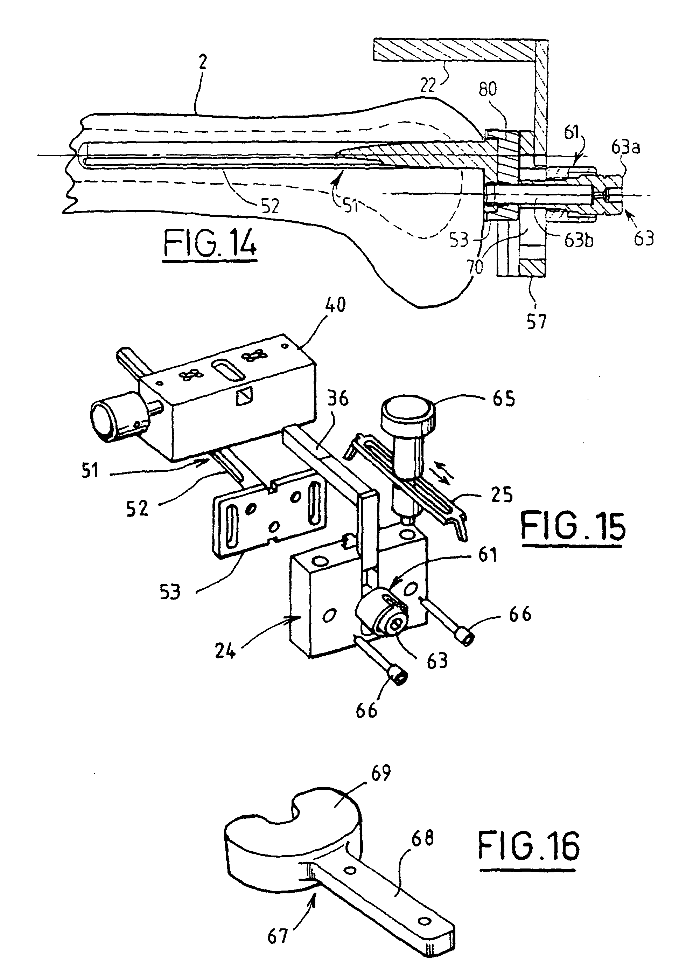

Le système de réglage et de blocage 61 comporte (figure 14) un écrou 61 enveloppant la tête 63a de la vis 63 et qui peut venir se visser sur la tige filetée 63b traversant une ouverture 70 du montant 57 , un coulisseau 80 et la plaque 53. Le coulisseau 80 peut être déplacé en translation dans une rainure 90 de forme complémentaire, par exemple en queue d'aronde du montant 57. Une fois la position de l'élément d'ancillaire 51 en translation sur le montant 57 réglée par coulissement de la vis 63 dans ce montant 57, la position ainsi choisie peut être bloquée par vissage de l'écrou 61 sur la tige filetée 63b jusqu'au blocage de cet écrou sur le montant 57.

Le coulisseau 80 est solidarisé avec la plaque 53 par la vis 63. Ce coulisseau étant d'autre part emboîté en translation dans la rainure 90, sa face inférieure ou postérieure 53a est parallèle au bras 23 d'appui sur la coupe tibiale 4. Il en résulte que la face 53a est toujours parallèle à la coupe tibiale proximale 4.

Le dispositif illustré à la figure 15 comprend un bloc 24 identique à celui de la figure 6 et un bloc 40 de coupe distale monté coulissant sur un bras 36 formant une potence elle-même réglable en position verticale sur le bloc 24. Ce dernier est également muni d'un système à molette 65 traversant le palpeur 25 monté coulissant par rapport à la molette 65. Le bloc 24 est pourvu d'un dispositif 61 de réglage et de blocage de la position de la plaque 53 de l'élément d'ancillaire 51 par rapport au bloc 24, lequel peut être relié à la plaque 53 par deux vis 66 et une vis 63 engagée dans le dispositif de blocage 61.

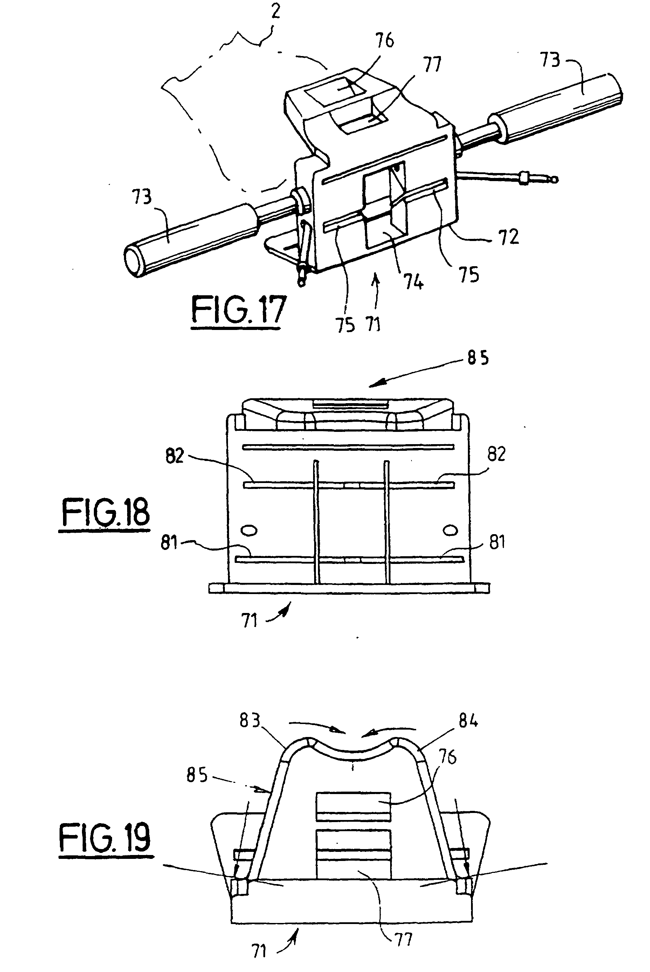

La figure 16 montre un espaceur 67 constituant un mode de réalisation industrielle de l'espaceur 32 (figure 7). L'espaceur 67 comporte une poignée 68 prolongée par une pièce semi-cylindrique 69 d'épaisseur appropriée.

Les figures 17 à 19 montrent une réalisation industrielle d'un bloc 71 de coupe des chanfreins de l'extrémité distale du fémur 2, après exécution des coupes antérieure, postérieure et distale. Le bloc 71 comprend un corps 72 sensiblement en U, muni de deux poignées latérales 73 de manutention et dans lequel peut venir s'engager le fémur 2. Dans la face extérieure du corps 72 sont ménagées à mi-hauteur, de part et d'autre d'une ouverture centrale 74, deux fentes 75 d'introduction de lames de coupe non représentées. Le bloc 71 est agencé de telle sorte que chaque fente 75 débouche, par des passages divergents sur deux fentes 81, 82 s'ouvrant sur la face intérieure du bloc, recevant le fémur 2: une fente basse 81, et une fente haute 82, la lame introduite par la fente 75 ayant l'une ou l'autre des inclinaisons correspondant à l'une des fentes 81 et 82. Deux autres ouvertures 76, 77 formées dans le corps 72 permettent également d'introduire dans celui-ci des lames de coupe non représentées pour la réalisation des autres chanfreins.

La partie antérieure 85 du bloc 71 présente un contour composé d'une première partie 83 correspondant au bord externe d'une prothèse côté droit, et d'une seconde partie 84 correspondant au bord externe d'une prothèse côté gauche.

Cet agencement présente l'avantage d'une mise en place optimale dans la direction médio-latérale.

Les coupes postérieures et distales obtenues au moyen de l'ancillaire et de la méthode chirurgicale qui peut être éffectuée avec l'ancillaire selon l'invention sont précises, et assurent une bonne tension ligamentaire. La tige-plaque 6 permet l'équilibrage ligamentaire en extension sans que la coupe distale ne soit faite.

Du fait de la précision de ces coupes, il est possible de réaliser les coupes secondaires constituées par les chanfreins sur l'extrémité fémorale au moyen du bloc unique 71 ou 79.

Selon la méthode chirurgicale décrite ci-dessus, on mesure l'écartement antéro-postérieur fémoral (AP) afin de déterminer la dimension correspondante de l'implant fémoral ; on procède à une coupe de la partie antérieure 29 puis de la partie postérieure 31 fémorale ; on mesure l'écart de flexion vrai (EF) entre le plan de coupe tibial et le plan de coupe fémoral postérieur (P), on détermine l'épaisseur ed de la coupe distale à effectuer en mesurant EF-E = ed, E étant la distance entre le plan de coupe tibiale 4 et la partie distale 18 du fémur 2 la plus proche ; et enfin on procède à la coupe de la partie distale 34 d'épaisseur ed.

En plus des avantages précédemment indiqués, le matériel 6 (tige-plaque) selon l'invention permet également d'effectuer toute la séquence opérationnelle consécutive à l'obtention de l'équilibre ligamentaire, lui-même obtenu à l'étape de la figure 2.

Ainsi l'invention évite d'avoir à procéder à une seconde coupe distale imprécise du fémur, ainsi que de replacer celui-ci en extension, ce qui représente un double avantage particulièrement appréciable.

Il convient de noter que les coupes antéro-postérieure des parties 29, 31 et distale de la partie 18 peuvent être exécutées simultanément ou consécutivement comme décrit ci-dessus, ce choix étant effectué par le chirurgien.

L'invention est susceptible de diverses variantes d'exécution. Ainsi l'inclinaison de la tige 8 sur la plaque 7 peut varier dans les limites indiquées ci-dessus, et la cale 9 peut être solidarisée de manière amovible avec la plaque 7 par tout moyen approprié, connu en soi.

## Revendications

1. Ancillaire pour la préparation de la posa d'une prothèse fémorale de genou comportant un plateau tibial et un implant fémoral, cet ancillaire comprenant une tige centro-médullaire (8,52) associée à un moyen permettant l'obtention d'un équilibre ligamentaire, **caractérisé en ce qu'**il comprend une plaque (7,53) ayant une épaisseur (d) au plus égale à celle d'un plateau tibial de la prothèse de genou, et à laquelle est fixée la tige (8,52) suivant une inclinaison (C) appropriée pour permettre de restituer l'interligne fémoral ou interligne virtuel avant usure de l'articulation, cette plaque étant destinée à venir prendre appui-sur un plan de coupe tibial (4) après insertion de sa tige dans le canal médullaire (10) du fémur afin de permettre l'obtention d'un équilibre ligamentaire et en vue d'un réglage ultérieur des écartements en extension (E) entre la coupe proximale du tibia (3) et l'extrémité distale (18) du fémur, et en flexion (EF) entre la coupe proximale du tibia (3) et la coupe postérieure du fémur, et **en ce qu'**il est pourvu d'une cale (9,55) adaptable sur ou pouvant être glissée en dessous de la plaque (7,53) et d'épaisseur variable, afin que l'épaisseur totale (E) de la plaque et de la cala soit égale à la distance entre le plan de coupe proximal tibial (4) et l'extrémité distale (18) du fémur (2) la plus proche dudit plan de coupe, après obtention de l'équilibre ligamentaire par distraction de l'articulation entre la plaque (7,53) et l'extrémité distale du fémur.

2. Ancillaire selon la revendication 1, **caractérisé en ce que** la cale (9) est munie de moyens de solidarisation amovible avec la plaque (7), par exemple grâce à un profil en U emboîtable sur la plaque et dont les branches (11) sont prolongées par des pattes (12) venant se loger dans des dégagements conjugués (13) formés sur les côtés de la plaque.

3. Ancillaire selon l'une des revendications 1 et 2 **caractérisé en ce que** la tige (8) a une inclinaison (C) dans le plan frontal sur la plaque de 3 degrés à 11 degrés environ en extension.

4. Ancillaire selon l'une des revendications 1 à 3, **caractérisé en ce que** la cale (9) présente à partir de l'un de ses bords un évidement médian (14) dans lequel peut venir s'insérer la tige (8) fixée à la plaque (6).

5. Ancillaire selon la revendication 1 **caractérisé en ce qu'**il est pourvu d'une cale (54) de forme allongée, ayant une largeur (I) sensiblement égale à la moitié de la longueur de la plaque (7), **en ce que** cette cale comporte une partie antérieure (56) d'épaisseur inférieure à celle de sa partie postérieure (55) et qui peut être glissée entre ladite plaque et la coupe proximale (4) du tibia, son épaisseur (d1) étant variable afin que l'épaisseur totale (E) de la plaque et de la cale soit égale à la distance entre le plan de coupe proximal tibial (4) et l'extrémité distale (18) du fémur (2) la plus proche dudit plan de coupe, après obtention de l'équilibre ligamentaire par distraction de l'articulation entre la plaque (7) et l'extrémité distale du fémur.

6. Ancillaire selon la revendication 1, comprenant un dispositif de mesure de la distance fémorale antéro-postérieure (A-P) de l'implant, **caractérisé en ce qu'**il comprend un montant (57) dans lequel peut coulisser un support (58) d'un bras (22) dont l'extrémité est adaptée pour venir s'appliquer sur l'extrémité distale antérieure du fémur (2), et un système de réglage (61) de la position en hauteur de la tige-plaque (7, 8) par rapport au montant, monté coulissant sur ce dernier et agencé pour pouvoir être solidarisé avec la plaque.

7. Ancillaire selon la revendication 1, comprenant un bloc (40) de coupe distale fémorale, un bras (36) de support de ce bloc monté coulissant sur un second bloc (24) pourvu d'un palpeur (25) ainsi que d'un système de réglage et de blocage de la position de la plaque (53) par rapport audit second bloc, ce système de réglage étant monté coulissant sur ledit second bloc et agencé pour pouvoir être solidarisé avec la plaque (53).

8. Ancillaire selon la revendication 6 ou 7, **caractérisé en ce que** le système de réglage (61) comporte une vis (63) traversant une ouverture (70) du montant (57), fixée à un coulisseau (80) mobile sur ledit montant et mécaniquement lié, par la vis (63) à la plaque (53), et un écrou (61) venant se visser sur la vis pour bloquer l'ensemble dans la position choisie sur le montant (57).

9. Ancillaire selon la revendication 8, **caractérisé en ce que** le coulisseau (80) est adapté pour coulisser dans une rainure complémentaire (90) du montant (57), cette rainure s'étendant perpendiculairement à un bras (23) d'appui du montant sur la coupe tibiale proximale (4), de telle sorte que la face postérieure (53a) de la plaque (53) soit parallèle audit bras d'appui (23).

10. Ancillaire selon la revendication 1, **caractérisé en ce qu'**il comprend un bloc de coupe (71) des chanfreins de l'extrémité distale du fémur (2) après exécution des coupes antérieure, postérieure et distale, et **en ce que** ce bloc comporte un corps (72) pourvu de poignées (73) de manutention, dans lequel sont ménagées des fentes (81, 82, 75) et des ouvertures (76, 77) d'introduction d'une lame de coupe.

11. Ancillaire selon la revendication 10, **caractérisé en ce que** le bloc de coupe (71) présente un contour composé d'une première partie (83) correspondant au bord externe d'une prothèse côté droit, et d'une seconde partie (84) correspondant au bord externe d'une prothèse côté gauche.

12. Ancillaire selon la revendication 1, **caractérisé en ce que** la plaque (7) comporte une face latérale (7a) perpendiculaire à sa face distale utilisée pour venir en appui sur le plan de coupe tibiale (4), cette face latérale (7a) constituant une surface de référence pour le plan de coupe tibiale (4) auquel elle doit être disposée parallèlement dans le plan frontal.

## Patentansprüche

1. Hilfsgerät zur Vorbereitung der Anbringung einer Kniefemoralprothese, die eine Tibialplatte und ein Femoralimplantat umfasst, wobei dieses Hilfsgerät eine Zentralmarkstange (8, 52) umfasst, die mit einem Mittel verbunden ist, das den Erhalt eines Gleichgewichts der Bänder ermöglicht, **dadurch gekennzeichnet, dass** es eine Platte (7, 53) umfasst, die eine Dicke (d) aufweist, die höchstens gleich jener einer Tibialplatte der Knieprothese ist, und an der die Stange (8, 52) mit einer entsprechenden Neigung (C) befestigt ist, um es zu ermöglichen, den Femoralzwischenraum oder virtuellen Zwischenraum vor dem Verschleiß des Gelenks wieder herzustellen, wobei diese Platte dazu bestimmt ist, nach dem Einsetzen ihrer Stange in den Markkanal (10) des Femur auf einer Tibialschnittebene (4) zur Auflage zu gelangen, um den Erhalt eines Gleichgewichts der Bänder zu ermöglichen, und im Hinblick auf eine spätere Einstellung der Abstände zwischen der Proximalschnittfläche der Tibia (3) und dem distalen Ende (18) des Femur bei Extension (E) und zwischen der Proximalschnittfläche der Tibia (3) und der posterioren Schnittfläche des Femur bei Flexion (EF), und dass es mit einem auf der Platte (7) derart anpassbaren Keil (9, 55) variabler Dicke versehen ist, dass die Gesamtdicke (E) der Platte und des Keils gleich dem Abstand ist, der zwischen der proximalen Tibialschnittebene (4) und dem der Schnittebene am nächsten liegendem distalen Ende (18) des Femur (2) nach Erhalt des Gleichgewichts der Bänder durch Distraktion des Gelenks zwischen der Platte (7, 53) und dem distalen Ende des Femur vorliegt.

2. Hilfsgerät nach Anspruch 1, **dadurch gekennzeichnet, dass** der Keil (9) mit lösbaren Mitteln zur Verbindung mit der Platte (7) versehen ist, beispielsweise mit einem U-Profil, das auf die Platte aufgesetzt werden kann und dessen Schenkel (11) durch Vorsprünge (12) verlängert sind, die in Aussparungen (13) entsprechender Form eingreifen, die auf den Seiten der Platte ausgebildet sind.

3. Hilfsgerät nach einem der Ansprüche 1 und 2, **dadurch gekennzeichnet, dass** die Stange (8) auf der Platte bei Extension in der Frontalebene eine Neigung (C) von etwa 3 Grad bis 11 Grad aufweist.

4. Hilfsgerät nach einem der Ansprüche 1 bis 3, **dadurch gekennzeichnet, dass** der Keil (9) ausgehend von einem seiner Ränder eine Mittelaussparung (14) aufweist, in die die an der Platte (6) befestigte Stange (8) eingesetzt wird.

5. Hilfsgerät nach Anspruch 1, **dadurch gekennzeichnet, dass** es mit einem Keil (54) langgestreckter Form versehen ist, der eine Breite (1) aufweist, die im Wesentlichen gleich der Hälfte der Länge der Platte (7) ist, dass dieser Keil einen anterioren Teil (56) umfasst, dessen Dicke geringer ist als jene seines posterioren Teils (55), und der zwischen die Platte und die proximale Schnittfläche (4) der Tibia eingeschoben werden kann, wobei seine Dicke (d1) derart variabel ist, dass die Gesamtdicke (E) der Platte und des Keils gleich dem Abstand ist, der zwischen der proximalen Tibialschnittfläche (4) und dem der Schnittebene am nächsten liegendem distalen Ende (18) des Femur (2) nach dem Erhalt des Bändergleichgewichts durch Distraktion des Gelenks zwischen der Platte (7) und dem distalen Ende des Femur vorliegt.

6. Hilfsgerät nach Anspruch 1, umfassend eine Vorrichtung zur Messung des anteroposterioren Femoralabstandes (A-P) des Implantats, **dadurch gekennzeichnet, dass** es einen Ständer (57), in dem eine Stütze (58) eines Arms (22) gleiten kann, dessen Ende derart ausgeführt ist, dass es an das distale anteriore Ende des Femur (2) angelegt werden kann, und ein System (61) zur Einstellung der Höhenposition der Stange-Platte (7, 8) in Bezug auf den Ständer umfasst, das gleitend auf diesem letztgenannten befestigt und derart ausgeführt ist, dass es mit der Platte verbunden werden kann.

7. Hilfsgerät nach Anspruch 1, umfassend einen Block (40) für den distalen Femoralschnitt, einen Stützarm (36) für diesen Block, der gleitend auf einem zweiten Block (24) befestigt ist, der mit einem Fühler (25) sowie einem System zur Einstellung und Feststellung der Position der Platte (53) in Bezug auf den zweiten Block versehen ist, wobei dieses Einstellsystem gleitend auf dem zweiten Block befestigt und derart angeordnet ist, dass es mit der Platte (53) verbunden werden kann.

8. Hilfsgerät nach Anspruch 6 oder 7, **dadurch gekennzeichnet, dass** das Einstellsystem (61) eine Schraube (63), die durch eine Öffnung (70) des Ständers (57) hindurchgeht und an einem auf dem Ständer beweglichen Schieber (80) befestigt ist, der mechanisch durch die Schraube (63) mit der Platte (53) verbunden ist, und eine Mutter (61) umfasst, die sich auf die Schraube schraubt, um die Einheit in der auf dem Ständer (57) gewählten Position festzustellen.

9. Hilfsgerät nach Anspruch 8, **dadurch gekennzeichnet, dass** der Schieber (80) derart ausgeführt ist, dass er in einer komplementären Nut (90) des Ständers (57) gleitet, wobei sich diese Nut senkrecht zu einen Stützarm (23) des Ständers auf der proximalen Tibialschnittfläche (4) erstreckt, sodass die posteriore Fläche (53a) der Platte (53) zu dem Stützarm (23) parallel ist.

10. Hilfsgerät nach Anspruch 1, **dadurch gekennzeichnet, dass** es einen Block (71) für den Schnitt der Kanten des distalen Endes des Femur (2) nach Ausführung der anterioren, posterioren und distalen Schnitte umfasst, und dass dieser Block einen mit Bediengriffen (73) versehenen Körper (72) umfasst, in dem Schlitze (81, 82, 75) und Öffnungen (76, 77) für die Einführung eines Schneidblattes vorgesehen sind.

11. Hilfsgerät nach Anspruch 10, **dadurch gekennzeichnet, dass** der Schneidblock (71) eine Kontur aufweist, die sich aus einem ersten Teil (83), der dem äußeren Rand einer rechtsseitigen Prothese entspricht, und einem zweiten Teil (84), der dem äußeren Rand einer linksseitigen Prothese entspricht, zusammensetzt.

12. Hilfsgerät nach Anspruch 1, **dadurch gekennzeichnet, dass** die Platte (7) eine Seitenfläche (7a) umfasst, die senkrecht auf ihrer distalen Fläche steht und verwendet wird, um auf der Tibialschnittfläche (4) zur Auflage zu gelangen, wobei diese Seitenfläche (7a) eine Referenzfläche für die Tibialschnittebene (4) darstellt, zu der sie in der Frontalebene parallel angeordnet werden muss.

## Claims

1. Ancillary device for the preparation of the fitting of a femoral knee prosthesis comprising a tibial plate and a femoral implant, this ancillary device comprising a centro-medullary rod (8, 52) associated with a means permitting a ligamentary equilibrium to be obtained, **characterised in that** it comprises a plate (7, 53) having a thickness (d) at most equal to that of a tibial plate of the knee prosthesis, and to which the rod (8, 52) is fixed at an appropriate inclination (C) to permit the femoral space or virtual space to be restored before wear of the joint, this plate being intended to bear on a tibial cutting plane (4) after insertion of the rod thereof into the medullary canal (10) of the femur in order to obtain a ligamentary equilibrium and with a view to subsequent adjustment of the deviations in extension (E) between the proximal cut of the tibia (3) and the distal end (18) of the femur, and in flexion (EF) between the proximal cut of the tibia (3) and the posterior cut of the femur, and **in that** it is provided with a wedge (9, 55) which can be adapted on the plate (7, 53) and has a variable thickness, so that the total thickness (E) of the plate and of the wedge is equal to the distance between the tibial proximal cutting plane (4) and the distal end (18) of the femur (2) closest to the said cutting plane, after ligamentary equilibrium is obtained by severance of the joint between the plate (7, 53) and the distal end of the femur.

2. Ancillary device as claimed in Claim 1, **characterised in that** the wedge (9) is equipped with means for removable connection to the plate (7), for example by virtue of a U-shaped profile which can be fitted on the plate and of which the arms (11) are extended by tabs (12) which are received in matching cut-outs (13) formed on the sides of the plate.

3. Ancillary device as claimed in one of Claims 1 and 2, **characterised in that** the rod (8) has an inclination (C) in the frontal plane with respect to the plate of about 3° to 11° in extension.

4. Ancillary device as claimed in one of Claims 1 to 3, **characterised in that** the wedge (9) has, starting from one of its edges, a median recess (14) into which the rod (8) fixed to the plate (6) can be inserted.

5. Ancillary device as claimed in Claim 1, **characterised in that** it is provided with a wedge (54) of elongate shape having a width (1) substantially equal to half the length of the plate (7), and **in that** this wedge includes an anterior part (56) with a thickness less than that of the posterior part (55) thereof and which can be slid between the said plate and the proximal cut (4) of the tibia, the thickness (d1) thereof being variable so that the total thickness (E) of the plate and of the wedge is equal to the distance between the tibial proximal cutting plane (4) and the distal end (18) of the femur (2) closest to the said cutting plane, after ligamentary equilibrium is obtained by severance of the joint between the plate (7) and the distal end of the femur.

6. Ancillary device as claimed in Claim 1, comprising a device for measuring the anteroposterior femoral distance (A-P) of the implant, **characterised in that** it comprises an upright (57) in which a support (58) of an arm (22) can slide, the end of which is adapted to be applied to the anterior distal end of the femur (2), and a system for adjustment (61) of the position of the rod/plate (7, 8) in terms of height with respect to the upright, mounted so as to slide on this latter and designed to be able to be connected to the plate (53).

7. Ancillary device as claimed in Claim 1, comprising a femoral distal cutting block (40), an arm (36) for supporting this block mounted so as to slide on a second block (24) provided with a probe (25) as well as a system for adjusting and locking the position of the plate (53) with respect to the said second block, this system of adjustment being mounted so as to slide on the said second block and designed to be able to be connected to the plate.

8. Ancillary device as claimed in Claim 6 or 7, **characterised in that** the system of adjustment (61) includes a screw (63) passing through an opening (70) in the upright (57), fixed to a slide (80) which is movable on the said upright and mechanically connected by the screw (63) to the plate (53), and a nut (61) which is screwed onto the screw in order to lock the assembly in the selected position on the upright (57).

9. Ancillary device as claimed in Claim 8, **characterised in that** the slide (80) is adapted to slide in a complementary groove (90) of the upright (57), this groove extending perpendicular to an arm (23) supporting the upright on the proximal tibial cut (4) in such a way that the posterior face (53a) of the plate (53) is parallel to the said support arm (23).

10. Ancillary device as claimed in Claim 1, **characterised in that** it comprises a block (71) for cutting the chamfers of the distal end of the femur (2) after execution of the anterior, posterior and distal cuts, and **in that** this block includes a body (72) which is provided with handles (73) for manipulation and in which slots (81, 82, 75) and openings (76, 77) are provided for the introduction of a cutting blade.

11. Ancillary device as claimed in Claim 10, **characterised in that** the cutting block (71) has a contour composed of a first part (83) corresponding to the outer edge of a right-side prosthesis, and second part (84) corresponding to the outer edge of a left-side prosthesis.

12. Ancillary device as claimed in Claim 1, **characterised in that** the plate (7) includes a lateral face (7a) perpendicular to its distal face used to bear on the tibial cutting plane (4), this lateral face (7a) constituting a reference surface for the tibial cutting plane (4) with respect to which it must be disposed parallel in the frontal plane.
